# EUROPEAN PATENT APPLICATION

(11) **EP 4 538 703 A1**
(43) Date of publication of application: **16.04.2025**
(21) Application number: 23819868.3
(22) Date of filing: 07.06.2023
(51) Int. Cl.: G01N 33/2028, G16C 20/30

(54) **DEVICE, METHOD, PROGRAM, AND SYSTEM FOR CALCULATING ALLOY COMPOSITION**

(30) Priority: 10.06.2022 JP 2022094234
(71) Applicant: Resonac Corporation, Tokyo 105-7325 (JP)
(72) Inventor: IKADAI, Hiroto, Tokyo 105-8518 (JP); IKEGAME, Ayami, Tokyo 105-8518 (JP); MINAMI, Takuya, Tokyo 105-8518 (JP); TAKEMOTO, Shimpei, Tokyo 105-8518 (JP); OKUNO, Yoshishige, Tokyo 105-8518 (JP)
(74) Representative: Strehl & Partner mbB
(86) International application number: PCT/JP2023/021143
(87) International publication number: WO 2023/238888

(57) **Abstract**

To easily calculate a comparable and contrastable indicator of a composition of an alloy for each manufacturing lot or for alloy particle. An alloy composition calculation apparatus includes an image acquisition unit configured to acquire element images of an alloy; a luminance identifying unit configured to identify, based on luminance values at identical coordinates in the element images, a point in a Cartesian coordinate system in which axes express luminance of the element images respectively; and a composition calculation unit configured to calculate a polar angle for the point, in polar coordinates, as a composition of the alloy.

## Description

### Technical Field

The present invention relates to an alloy composition calculation apparatus, a method, a program, and a system.

### Background

Conventionally, methods for analyzing elemental compositions of alloys have been known. For example, in Non-Patent Document 1, an alloy is statistically analyzed from an element mapping image that is obtained by STEM (scanning transmission electron microscopy)-XEDS (X-ray energy-dispersive spectroscopy).

### Related-Art Document

### Non-Patent Document

Non-Patent Document 1: Xuan Quy Tran, Yoshiki Kono, Tomokazu Yamamoto, Kohei Kusada, Hiroshi Kitagawa, and Syo Matsumura, "Statistical Evaluation of the Solid-Solution State in Ternary Nanoalloys," THE JOURNAL OF PHYSICAL CHEMISTRY C, 2020,124,21843-21852

### Summary

### Problem to be solved by the invention

In the industrial use of alloys, there is a demand for simple indications that can quantitatively control quality between alloy particles and between manufacturing lots of alloys. However, although Non-Patent Document 1 statistically analyzes images of compositions of alloys, it does not describe or suggest indications, of the compositions of the alloys, that are appropriate for quantitative comparison between alloy particles and between manufacturing lots.

An object of the present invention is to provide a simple indicator useful for comparison and contrast of individual manufacturing lots or alloy particles, as a composition of an alloy.

### Means for Solving the Problem

[1] An alloy composition calculation apparatus includes:
   an image acquisition unit configured to acquire element images of an alloy;
   a luminance identifying unit configured to identify, based on luminance values at identical coordinates in the element images, a point in a Cartesian coordinate system in which axes express luminance of the element images respectively; and
   a composition calculation unit configured to calculate a polar angle for the point, in polar coordinates, as a composition of the alloy.
[2] In the alloy composition calculation apparatus in [1], the composition calculation unit may be configured to calculate the composition of the alloy for each particle in the alloy, or for each image of the alloy.
[3] In the alloy composition calculation apparatus in [1] or [2], the composition calculation unit may be configured to calculate the composition of the alloy based on a distribution of polar angles in the calculated polar coordinates.
[4] In the alloy composition calculation apparatus in [3], the alloy composition may be defined by at least one of a standard deviation, a median value, an average value, or skewness.
[5] The alloy composition calculation apparatus in any one of [1] to [4] may further include:
   a visualization unit configured to visualize the composition of the alloy based on polar angles in the calculated polar coordinates.
[6] In the alloy composition calculation apparatus in [5], the visualization unit is configured to provide a distribution of the polar angles in the polar coordinates in a histogram.
[7] In the alloy composition calculation apparatus in [5], the visualization unit may be configured to visualize a distribution of luminance values on each of the element images.
[8] A method executed by an alloy composition calculation apparatus, includes:
   acquiring element images of an alloy;
   identifying, based on luminance values at identical coordinates in the element images, a point in a Cartesian coordinate system in which axes express luminance of the element images respectively; and
   calculating a polar angle for the point, in polar coordinates, as a composition of the alloy.
[9] A program cause an alloy composition calculation apparatus to function as:
   an image acquisition unit configured to acquire element images of an alloy;
   a luminance identifying unit configured to identify, based on luminance values at identical coordinates in the element images, a point in a Cartesian coordinate system in which axes express luminance of the element images respectively; and
   a composition calculation unit configured to calculate a polar angle for the point, in polar coordinates, as a composition of the alloy.
[10] A system includes:
   an alloy composition calculation apparatus;
   an alloy observation apparatus; and
   an alloy manufacturing apparatus,
   wherein the alloy composition calculation apparatus includes
      an image acquisition unit configured to acquire element images of an alloy,
      a luminance identifying unit configured to identify, based on luminance values at identical coordinates in the element images, a point in a Cartesian coordinate system in which axes express luminance of the element images respectively, and
      a composition calculation unit configured to calculate a polar angle for the point, in polar coordinates, as a composition of the alloy, and
   wherein the alloy manufacturing apparatus is configured to manufacture the alloy based on a result of calculation by the alloy composition calculation apparatus.

### Effects of the Invention

According to the present invention, an easily comparable indicator of a composition of an alloy can be calculated.

### Brief Description of the Drawings

[FIG. 1] FIG. 1 is an overall structural diagram according to one embodiment of the present invention.
[FIG. 2] FIG. 2 is a hardware structural diagram of an alloy composition calculation apparatus according to one embodiment of the present invention.
[FIG. 3] FIG. 3 is a functional block diagram of the alloy composition calculation apparatus according to one embodiment of the present invention.
[FIG. 4] FIG. 4 is a diagram for describing the calculation of an alloy composition for each alloy particle and the calculation of an alloy composition for each alloy image according to one embodiment of the present invention.
[FIG. 5] FIG. 5 is a flowchart of the overall process according to one embodiment of the present invention.
[FIG. 6] FIG. 6 is a flowchart of preprocessing according to one embodiment of the present invention.
[FIG. 7] FIG. 7 shows an example of each element image of the alloy according to one embodiment of the present invention.
[FIG. 8] FIG. 8 shows an example of an image during preprocessing according to one embodiment of the present invention.
[FIG. 9] FIG. 9 is a flowchart of an alloy composition calculation process according to one embodiment of the present invention.
[FIG. 10] FIG. 10 is a diagram for describing polar coordinates according to one embodiment of the present invention.
[FIG. 11] FIG. 11 shows an example of a screen according to one embodiment of the present invention.
[FIG. 12] FIG. 12 shows an example of a screen according to one embodiment of the present invention.
[FIG. 13] FIG. 13 shows an example of a screen according to one embodiment of the present invention.
[FIG. 14] FIG. 14 shows an example of a screen according to one embodiment of the present invention.
[FIG. 15] FIG. 15 shows an example of a screen according to one embodiment of the present invention.
[FIG. 16] FIG. 16 shows an example of a screen according to one embodiment of the present invention.
[FIG. 17] FIG. 17 shows an example of a screen according to one embodiment of the present invention.
[FIG. 18] FIG. 18 shows an example of a screen according to one embodiment of the present invention.
[FIG. 19] FIG. 19 shows an example of a screen according to one embodiment of the present invention.

### Mode for Carrying out the Invention

Embodiments of the present invention will be described below with reference to the accompanying drawings. In the present specification and the drawings, components having substantially the same functional configuration are denoted by the same reference numerals, and accordingly, redundant description is omitted.

### <Overall Configuration>

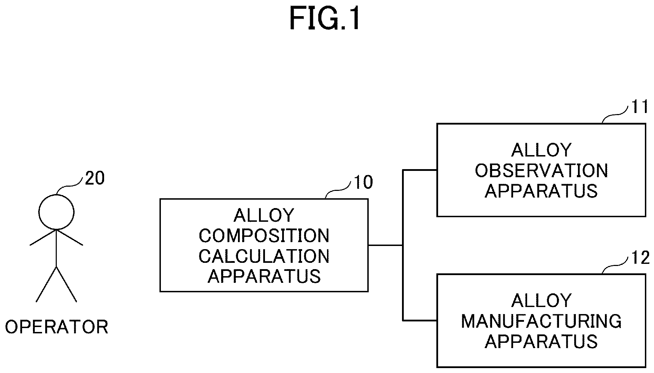

FIG. 1 is an overall structural diagram according to one embodiment of the present invention. An alloy composition calculation apparatus 10 is operated by an operator 20. The alloy composition calculation apparatus 10 can transmit and receive data with respect to both an alloy observation apparatus 11 and an alloy manufacturing apparatus 12.

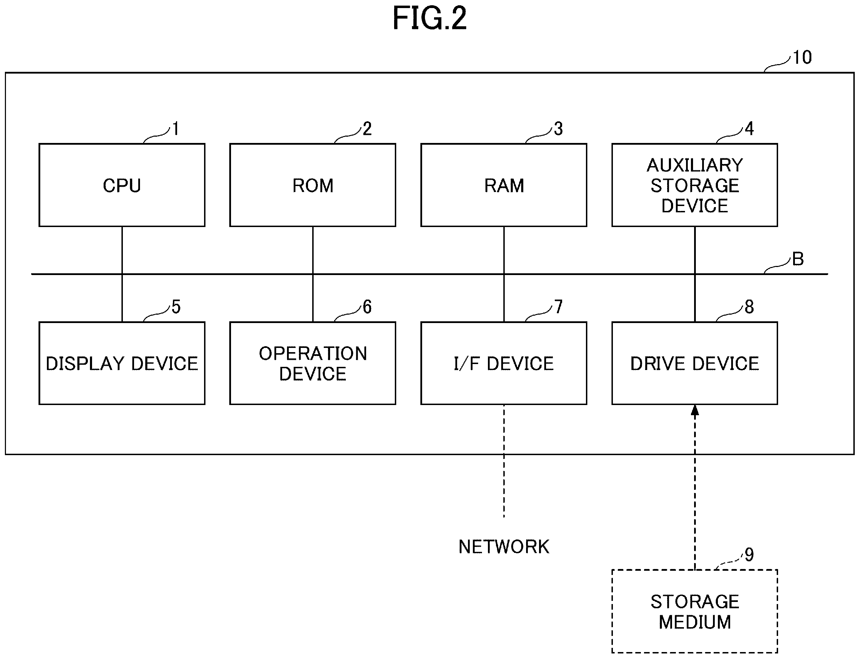

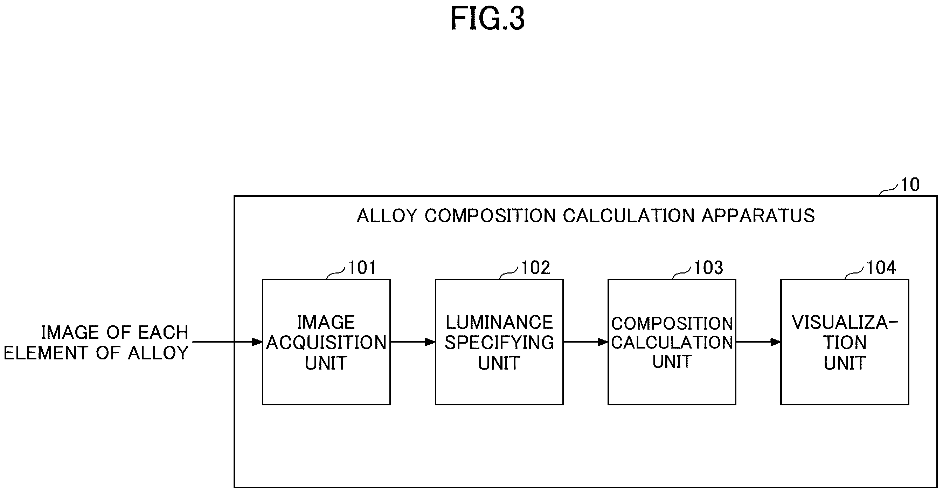

The alloy composition calculation apparatus 10 is an apparatus for calculating a composition of an alloy. The alloy composition calculation apparatus 10 is implemented by one or more computers (for example, a personal computer to be operated by the operator 20 or a combination of the personal computer to be operated by the operator 20 and a server). The alloy composition calculation apparatus 10 will be described in detail below with reference to FIG. 2 and FIG. 3.

The alloy observation apparatus 11 is an apparatus for acquiring an image of the alloy. For example, the alloy observation apparatus 11 is an electron microscope such as a scanning electron microscope (SEM) or a scanning transmission electron microscope (STEM) .

The alloy manufacturing apparatus 12 is an apparatus for manufacturing the alloy.

One embodiment of the present invention is a system including the alloy composition calculation apparatus 10, the alloy observation apparatus 11, and the alloy manufacturing apparatus 12. In the embodiment of the present invention, the alloy manufacturing apparatus 12 manufactures the alloy, the alloy observation apparatus 11 acquires an image of the alloy (that is, the alloy manufactured by the alloy manufacturing apparatus 12), and the alloy composition calculation apparatus 10 calculates the composition of the alloy (that is, the alloy manufactured by the alloy manufacturing apparatus 12). Thereafter, the alloy manufacturing apparatus 12 can manufacture the alloy based on the result of the calculation that is performed by the alloy composition calculation apparatus 10 (for example, manufacturing conditions are changed based on a result of calculation that is performed by the alloy composition calculation apparatus 10).

### <Hardware Configuration>

FIG. 2 is a hardware structural diagram of the alloy composition calculation apparatus 10 according to one embodiment of the present invention. The alloy composition calculation apparatus 10 includes a central processing unit (CPU) 1, a read only memory (ROM) 2, and a random access memory (RAM) 3. The CPU 1, the ROM 2, and the RAM 3 constitute a computer. The alloy composition calculation apparatus 10 may also include an auxiliary storage device 4, a display device 5, a control device 6, an interface (I/F) device 7, and a drive device 8. The hardware components of the alloy composition calculation apparatus 10 are connected to one another via a bus B.

The CPU 1 is an arithmetic device for executing various programs that are installed in the auxiliary storage device 4.

The ROM 2 is a nonvolatile memory. The ROM 2 functions as a main storage device for storing various programs and data necessary for the CPU 1 to execute the various programs installed in the auxiliary storage device 4. Specifically, the ROM 2 functions as a main storage device for storing boot programs such as a BIOS (Basic Input/Output System) and an EFI (Extensible Firmware Interface).

The RAM 3 is a volatile memory such as a DRAM (Dynamic Random Access Memory) and an SRAM (Static Random Access Memory). The RAM 3 functions as a main storage device for providing a work area where the programs installed in the auxiliary storage device 4 are expanded when executed by the CPU 1.

The auxiliary storage device 4 is an auxiliary storage device for storing various programs and information that is used when the various programs are executed.

The display device 5 is a display device for displaying an input/output status, operations, and the like of the programs for the alloy composition calculation apparatus 10.

The control device 6 is an input device that is used for an administrator of the alloy composition calculation apparatus 10 to input various instructions to the alloy composition calculation apparatus 10.

The I/F device 7 is a communication device for connecting to a network and communicating with other devices.

The drive device 8 is a device in which a storage medium 9 is set. The storage medium 9 includes a medium for recording information optically, electrically, or magnetically, such as a CD-ROM, a flexible disk, or a magneto-optical disk. The storage medium 9 may also include a semiconductor memory for electrically recording information, such as an EPROM (Erasable Programmable Read Only Memory) or a flash memory.

The various programs to be installed in the auxiliary storage device 4 are installed by, for example, setting a distributed storage medium 9 in the drive device 8 and reading the various programs recorded in the storage medium 9 through the drive device 8. Alternatively, the various programs to be installed in the auxiliary storage device 4 may be installed through downloading the programs from the network via the I/F device 7.

### <Functional Block>

FIG. 3 is a functional block diagram of the alloy composition calculation apparatus 10 according to one embodiment of the present invention. The alloy composition calculation apparatus 10 can include an image acquisition unit 101, a luminance identifying unit 102, a composition calculation unit 103, and a visualization unit 104. By executing one or more programs, the alloy composition calculation apparatus 10 can function as the image acquisition unit 101, the luminance identifying unit 102, the composition calculation unit 103, and the visualization unit 104.

The image acquisition unit 101 acquires an image including each element image (specifically, two or more element images) of the alloy. For example, the image acquisition unit 101 acquires the image (for example, an image including an elemental mapping image that is acquired using an EDS (X-ray energy-dispersive spectroscopy) or the like in an SEM (scanning electron microscope), an STEM (scanning transmission electron microscope), or the like), of each element of the alloy, acquired by an electron microscope.

As the alloy, any alloy may be used. The two or more element images may all be images of metallic elements, or may be a combination of an image of a non-metallic element and an image of at least one metallic element.

As examples of one or more alloys targeted by the alloy composition calculation apparatus 10 according to the present invention, alloy particles or the like such as a Pt-Co catalyst used in a combustion battery catalyst; a Pt-Rh catalyst used in an automobile exhaust gas device or the like; and various alloy catalysts used for the production of organic molecular materials and the decomposition treatment of organic molecules are used. A catalyst containing an element other than a metal can be used as a target as long as each element contained in the catalyst is an element for which an image can be acquired.

The luminance identifying unit 102 identifies a point in a Cartesian coordinate system with each axis expressing the luminance of a corresponding element image, based on the respective luminance values at the same coordinates in element images (that is, the luminance values of pixels located at the same coordinates in element images) acquired by the image acquisition unit 101.

The composition calculation unit 103 calculates a polar angle in polar coordinates for the point identified by the luminance identifying unit 102, to define a given alloy composition. The composition calculation unit 103 can calculate the alloy composition for each alloy particle or for each alloy image.

The composition calculation unit 103 can also calculate, for the alloy composition, both the polar angle, in the polar coordinates, for the point, and a distance of the above point from the origin in the polar coordinates.

The visualization unit 104 visualizes the alloy composition based on the polar angle, in the polar coordinates, calculated by the composition calculation unit 103. For example, the visualization unit 104 visualizes the distribution of polar angles in the polar coordinates with a histogram.

The composition calculation unit 103 can also calculate the alloy composition based on the calculated distribution of the polar angles in the polar coordinates. For example, the composition calculation unit 103 can calculate, for the alloy composition, a standard deviation, a median value, an average value, skewness, and the like, based on the distribution of the polar angles in the polar coordinates.

### <<Calculation of alloy composition for each alloy particle, and calculation of alloy composition for each alloy image>>

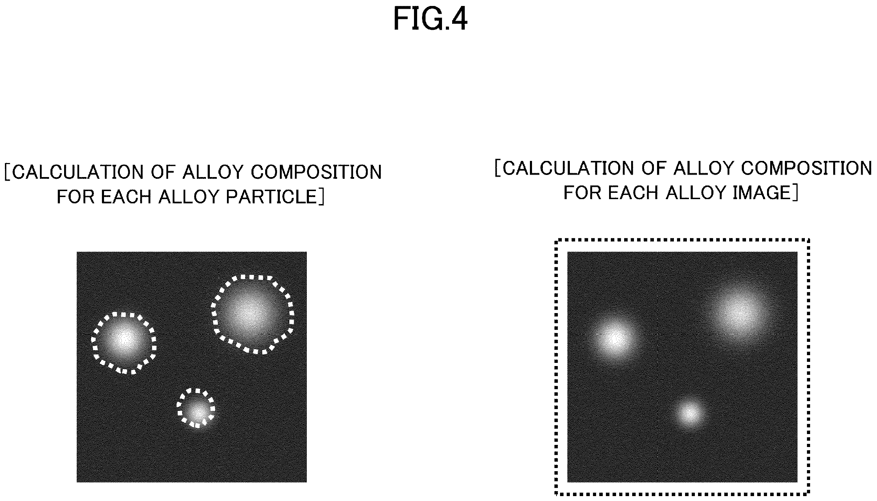

As described above, the alloy composition calculation apparatus 10 can calculate the alloy composition for each alloy particle or for each alloy image. Detailed description will be provided below with reference to FIG. 4.

FIG. 4 is a diagram for describing both the calculation of the alloy composition for each alloy particle and calculation of the alloy composition for each alloy image according to one embodiment of the present invention.

As indicated by [calculation of alloy composition for each alloy particle] on the left side of FIG. 4, the alloy composition calculation apparatus 10 can calculate an alloy composition for each particle (a region enclosed by a given dotted line in FIG. 4). The alloy composition calculation apparatus 10 calculates a polar angle in polar coordinates based on the luminance of each pixel in the particle (the region enclosed by the given dotted line in FIG. 4).

As indicated by [calculation of alloy composition for each alloy image] on the right side of FIG. 4, the alloy composition calculation apparatus 10 can calculate an alloy composition for each image (a region enclosed by a dotted line in FIG. 4). The alloy composition calculation apparatus 10 calculates a polar angle in polar coordinates based on the luminance of each pixel in the image (the region enclosed by the dotted line in FIG. 4).

In the following, the calculation of the alloy composition for each alloy particle will be mainly described, but the same approach applies to the calculating of the alloy composition for each alloy image.

### <Method>

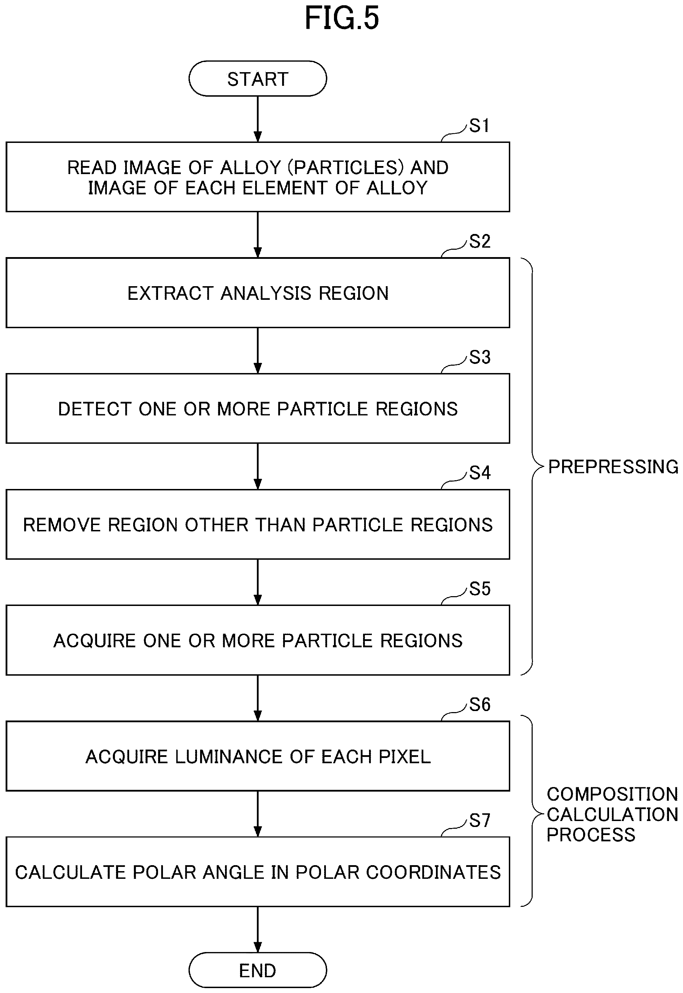

FIG. 5 is a flowchart of the entire process according to one embodiment of the present invention.

In step 1 (S1), the image acquisition unit 101 reads an image (alloy (particle) image) of alloy particles and each element image of an alloy.

In steps 2 to 5, the images acquired in S1 are preprocessed.

In step 2 (S2), the image acquisition unit 101 extracts a region (analysis region) to be analyzed from each of the images read in S1.

In step 3 (S3), the image acquisition unit 101 detects one or more regions of particles (particle regions) in the analysis region extracted in S2.

In step 4 (S4), the image acquisition unit 101 removes a region other than the particle regions detected in S3, from the analysis region in S2.

In step 5 (S5), the image acquisition unit 101 acquires the particle regions.

In steps 6 and 7, a process of calculating an alloy composition is performed.

In step 6 (S6), the luminance identifying unit 102 acquires the luminance of each pixel.

In step 7 (S7), the composition calculation unit 103 calculates a polar angle in the polar coordinates.

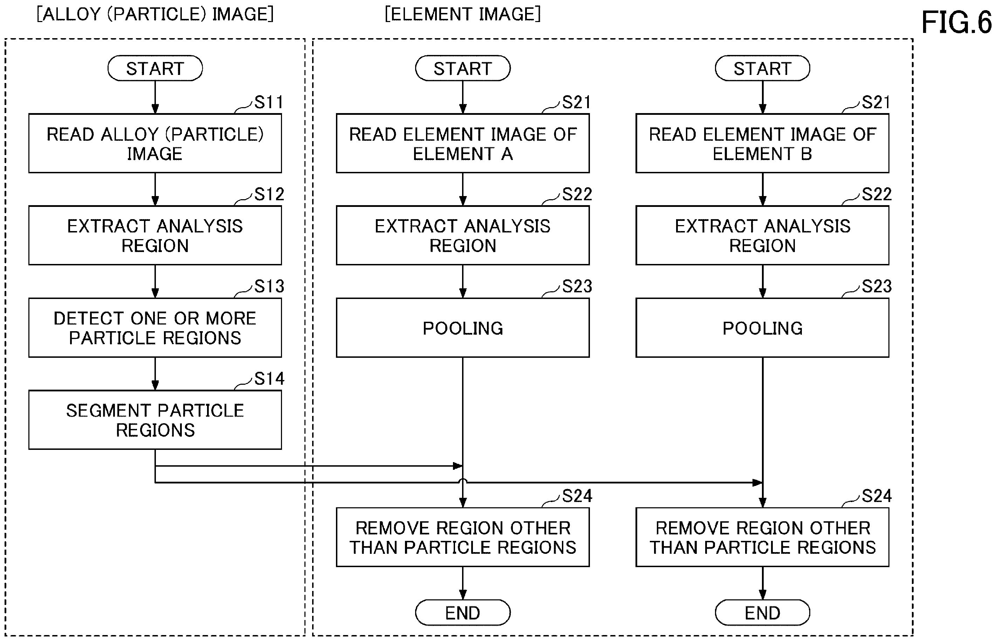

FIG. 6 is a flowchart of the preprocessing according to one embodiment of the present invention.

### <<Process for Alloy (Particle) Image>>

In step 11 (S11), the image acquisition unit 101 reads an image (alloy (particle) image) of one or more alloy particles.

In step 12 (S12), the image acquisition unit 101 extracts a region (analysis region) to be analyzed from the image read in S11.

In step 13 (S13), the image acquisition unit 101 detects one or more particle regions in the image. Specifically, the image acquisition unit 101 binarizes the image (e.g., using simple binarization, Otsu's binarization, or adaptive binarization) to perform erosion, dilation, and/or noise removal (note that the order of erosion, dilation, and noise removal is not fixed. The erosion, the dilation, and/or the noise removal may be omitted), and detects one or more particle regions.

In step 14 (S14), the image acquisition unit 101 segments regions (particle regions) of particles in the image for each particle.

### <Process for Elemental Images>

In each step 21 (S21), the image acquisition unit 101 reads an element image (for example, each of an element image of an element A and an element image of an element B).

In each step 22 (S22), the image acquisition unit 101 extracts a region (analysis region) to be analyzed from the image read in S21.

In each step 23 (S23), the image acquisition unit 101 performs a pooling process (e.g., average pooling, max pooling, or the like) on the analysis region extracted in S22. For example, the image acquisition unit 101 can set an average value, a maximum value, or the like of pixel values of n×n pixels, as a pixel value for each pixel in a corresponding n×n region.

In each step 24 (S24), the image acquisition unit 101 removes, from the element image, a region other than the one or more particle regions, based on information of the particle regions in the alloy (particle) image in S14.

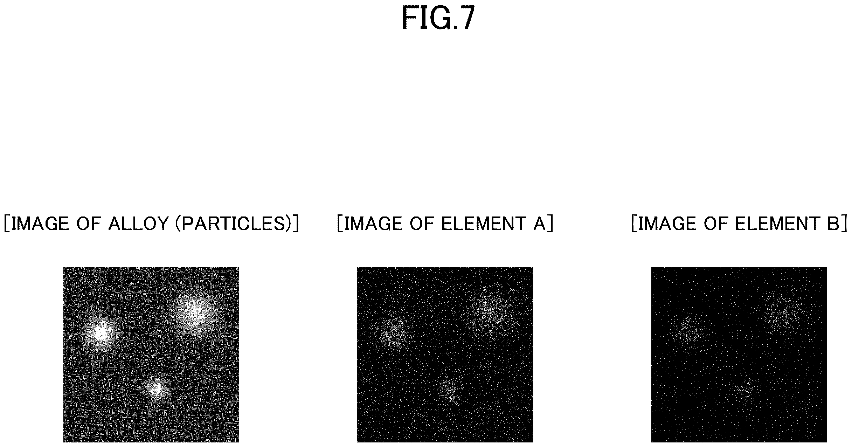

FIG. 7 shows an example of each element image of the alloy according to one embodiment of the present invention. [Image of alloy (particles)] shows an example of the image of the alloy particles. [Image of element A] shows an example of the element image of the element A in the alloy. [Image of element B] shows an example of the element image of the element B in the alloy. [Image of alloy (particles)], [Image of element A], and [Image of element B] are obtained using an electron microscope (for example, an SEM (scanning electron microscope) image, a STEM (scanning transmission electron microscope) image, or the like).

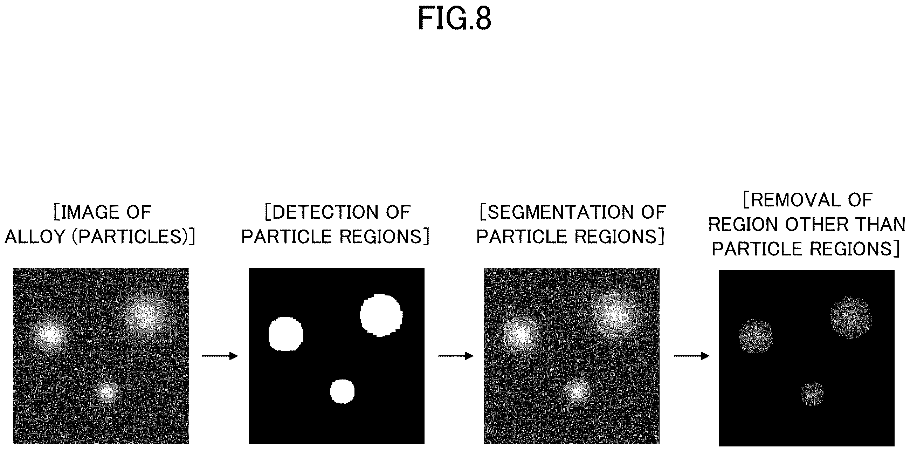

FIG. 8 shows an example of the image during preprocessing according to one embodiment of the present invention.

[Image of alloy (particles)] shows an example of the image of the alloy particle that is read in S11 of FIG. 6 and from which the analysis region has been extracted in S12.

[Detection of particle regions] shows an example of the image in which the particle regions have been detected in S13 of FIG. 6.

[Segmentation of particle regions] shows an example of an image in which the particle regions have been segmented in S14 of FIG. 6.

[Removal of region other than particle regions] shows an example of an image from which the region other than the particle regions has been removed in S24 of FIG. 6.

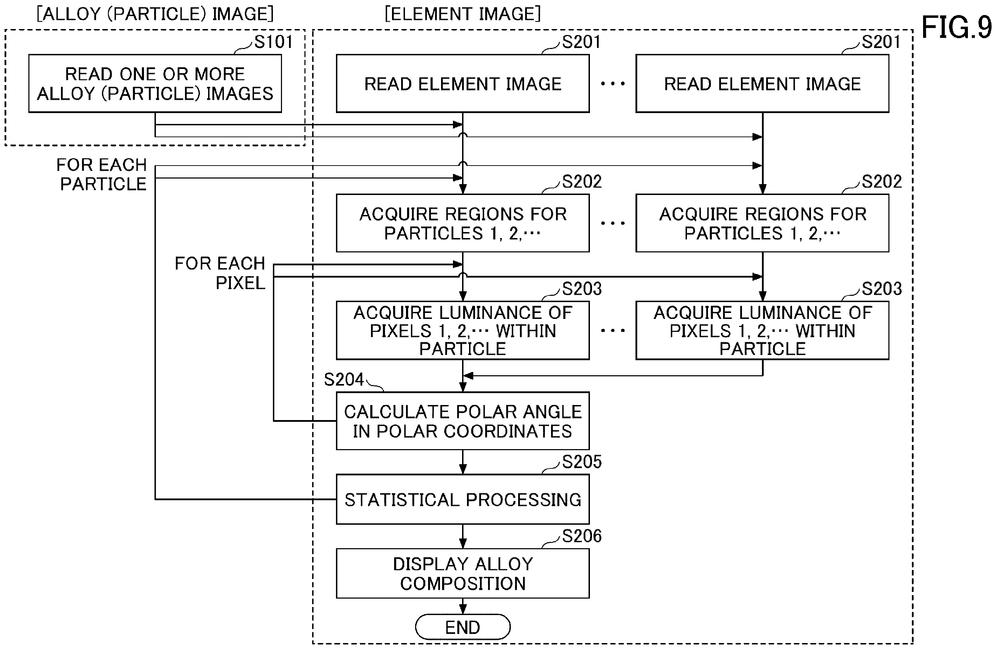

FIG. 9 is a flowchart of an alloy composition calculation process according to one embodiment of the present invention.

### <<Process for Alloy (Particle) Image>>

In step 101 (S101), the luminance identifying unit 102 reads the image (alloy (particle) image) of the alloy particles having individually segmented particle regions as obtained in the above preprocessing S14.

### <<Process for Element Images>>

In each step 201 (S201), the luminance identifying unit 102 reads an element image (for example, each of the element image of the element A and the element image of the element B), as obtained in the above preprocessing S24.

In each step 202 (S202), the luminance identifying unit 102 acquires the region of a particle (which is designated as Particle 1) in the element image, based on the image of the alloy particles (alloy (particle) image) having the individually segmented particle regions.

In each step 203 (S203), the luminance identifying unit 102 acquires the luminance of a pixel (which is designated as Pixel 1) in the region of the particle (which is designated as Particle 1) acquired in S202.

In step 204 (S204), the composition calculation unit 103 calculates a polar angle in the polar coordinates based on the pixel luminance of the particle region in each element image acquired in S202. Thereafter, the process returns to S203, and the luminance of a subsequent pixel (which is designated as Pixel 2) is acquired. With this approach, the process is repeated until polar angles in the polar coordinates are acquired for all pixels in the region of the particle.

In step 205 (S205), the composition calculation unit 103 performs a statistical process based on the distribution of the polar angles in the polar coordinates as calculated in S204. In the statistical process, statistical analysis is performed on the distribution of the polar angles in the polar coordinates, across the region of the particle (which is designated as Particle 1), and an alloy composition of the particle is calculated. For the alloy composition to be calculated, at least one of an average value, a standard deviation, a median value, or skewness is calculated. Thereafter, the process returns to S202, and the distribution of polar angles in the polar coordinates in a region of a subsequent particle (which is designated as Particle 2) is acquired. With this approach, the process is repeated until the statistical process is performed on the distribution of polar angles in the polar coordinates of all particle regions.

In step 206 (S206), the composition calculation unit 103 displays the alloy composition calculated in S205, for each particle.

### <Polar Coordinates>

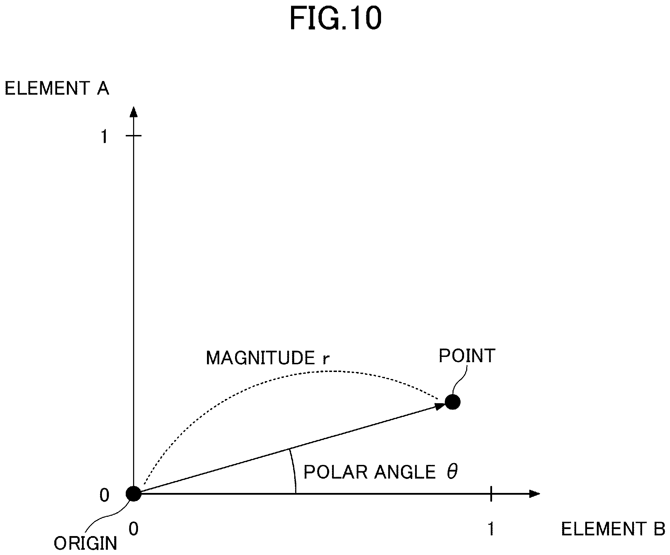

FIG. 10 is a diagram for describing the polar coordinates according to one embodiment of the present invention. A case of an alloy having two elements (with use of a two-dimensional polar coordinate system) will be described, but the same approach applies to alloys having three or more elements (with use of a three-dimensional or higher polar coordinate system).

FIG. 10 shows a Cartesian coordinate system in which the vertical axis is an axis of the luminance of the element image of the element A in the alloy, and the horizontal axis is an axis of the luminance of the element image of the element B in the alloy. A "point" is a point at which luminance values at the same coordinates on both the element image of the element A and the element image of the element B (that is, the luminance values of pixels located at the same coordinates on the respective element images) are plotted. An angle formed by a vector from the origin to the "point" and a ray (which corresponds to an axis for the luminance of the element image of the element B in the alloy, i.e., the horizontal axis) is expressed as a polar angle θ. A distance (a distance between the origin and the "point") from the origin in polar coordinates is expressed by r. For all pixels, polar angles θ in the polar coordinates, and distances r from the origin in the polar coordinates are calculated. One or more pixels where the luminance of the element image of the element A and the luminance of the element image of the element B are both zero are excluded. Here, luminance values are scaled to a maximum of 1.

The polar angle θ in the polar coordinates indicates a difference in the luminance between element images, that is, a bias in elements. The distance r from the origin in the polar coordinates corresponds to a luminance magnitude, that is, an element amount.

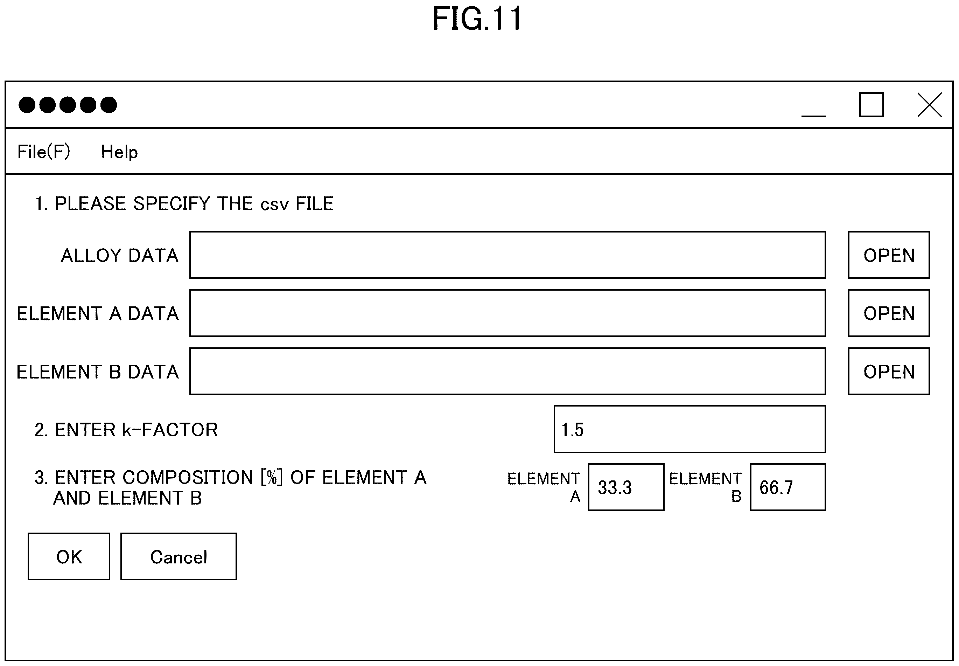

Examples of screens of an application program according to one embodiment of the present invention will be described below with reference to FIGS. 11 to 18.

### [Initial Screen]

FIG. 11 shows an example of a screen according to one embodiment of the present invention. FIG. 11 is a screen in which a user of an application program selects alloy data and one or more pieces of element data, enters a correction value (k-factor), and enters one or more setting values.

As the alloy data and each piece of element data, numerical data (for example, a csv file format) representing the luminance (X-ray intensity) of each pixel may be used, or image data such as a bitmap may be used.

The correction value (k-factor) is a correction value for converting the luminance (X-ray intensity) into a composition.

The setting values relate to a composition (in an example of FIG. 11, the element A accounts for 33.3% and the element B accounts for 66.7%.) for the alloy data that is selected by the user. For example, a design composition is entered. The luminance of each element image is calibrated based on a corresponding setting value.

### [Parameter Setting Screen (Binarization)]

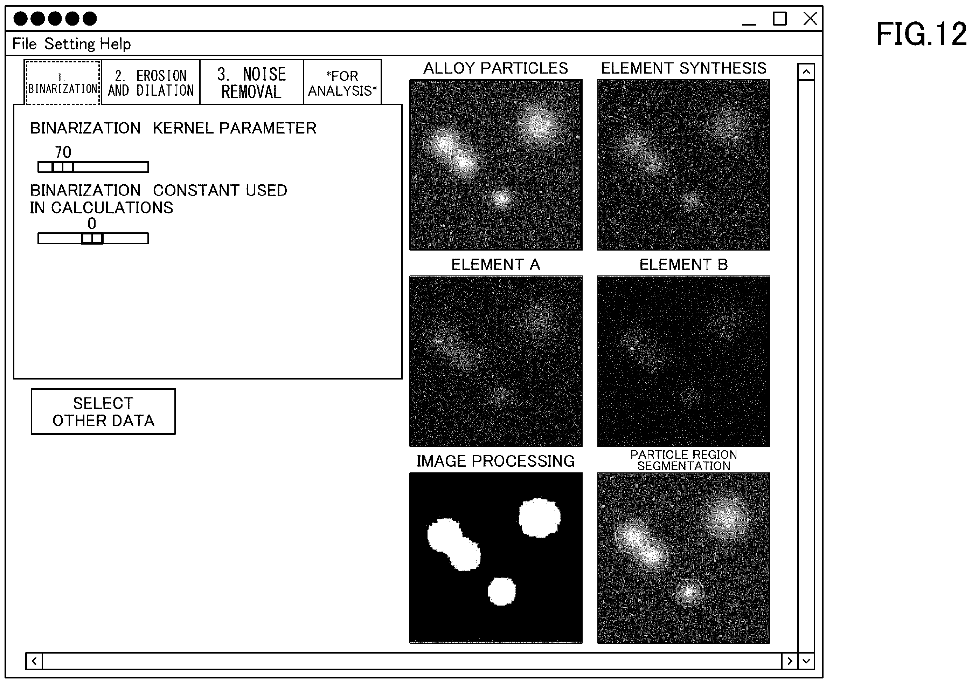

FIG. 12 shows an example of a screen according to one embodiment of the present invention. FIG. 12 shows the screen for the user of the application program to configure binarization settings. The user can set (for example, change initial values of) one or more parameters related to the binarization. Instead of the user entering various setting values, the alloy composition calculation apparatus 10 may determine one or more optimum values.

### [Parameter Setting Screen (Erosion and Dilation)]

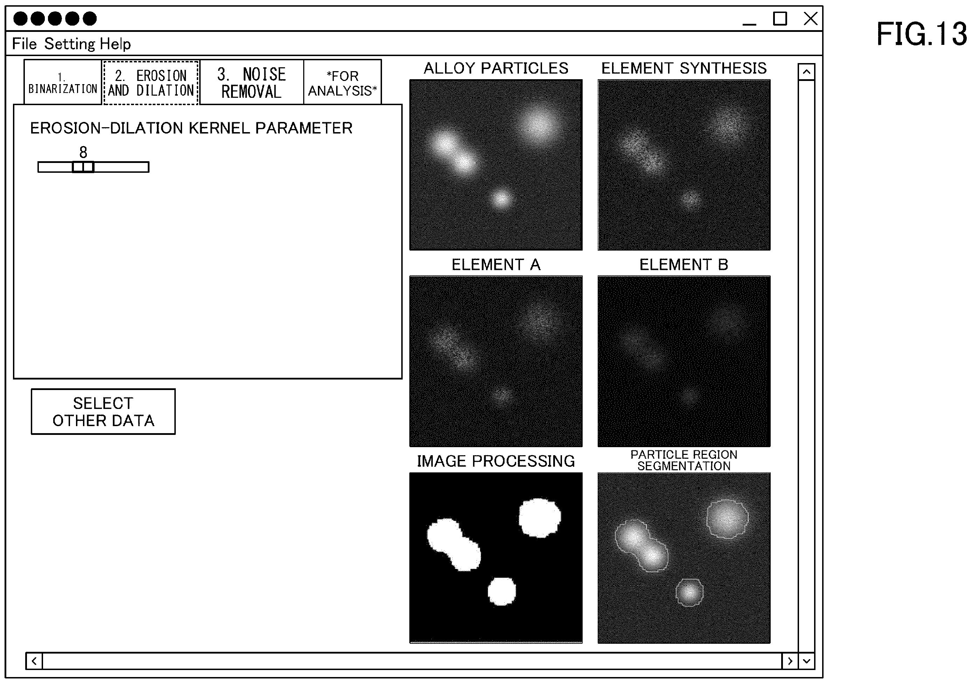

FIG. 13 shows an example of a screen according to one embodiment of the present invention. FIG. 13 shows the screen for the user of the application program to configure erosion and dilation settings. The user can set (for example, change initial values of) parameters related to the erosion and dilation. Instead of the user entering various setting values, the alloy composition calculation apparatus 10 may determine one or more optimum values.

### [Parameter Setting Screen (Noise Removal)]

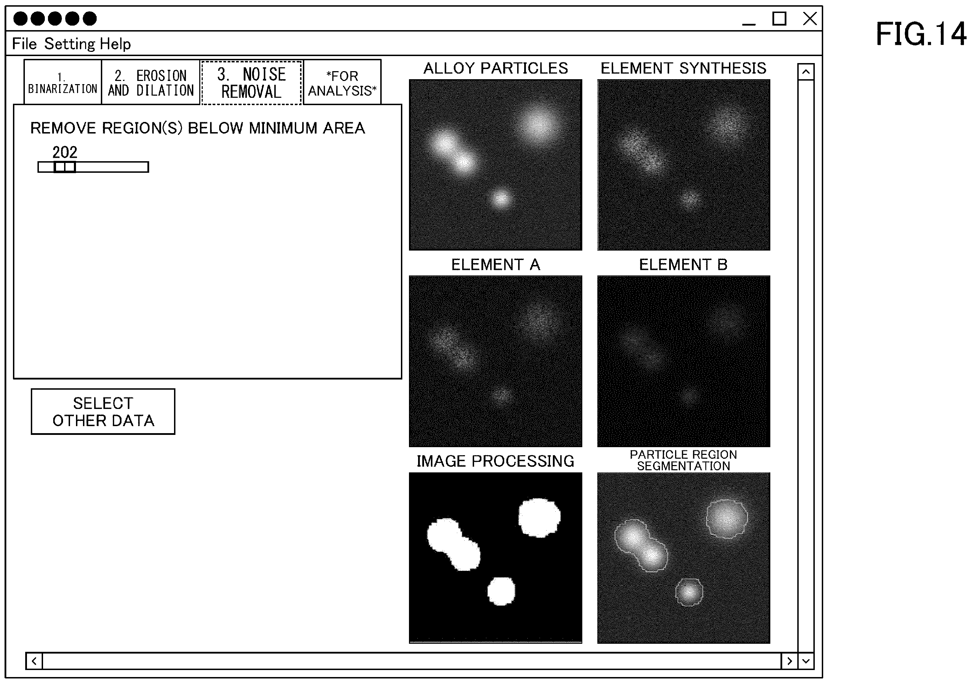

FIG. 14 shows an example of a screen according to one embodiment of the present invention. FIG. 14 shows the screen for the user of the application program to configure noise removal settings. The user can set (for example, change initial values of) parameters related to the noise removal. When there is a small region that is appropriate to treat as noise, the region can be excluded from an analysis target as a region having a specified area or less. Instead of the user entering various setting values, the alloy composition calculation apparatus 10 may determine one or more optimum values.

### [Parameter Setting Screen (for Analysis)]

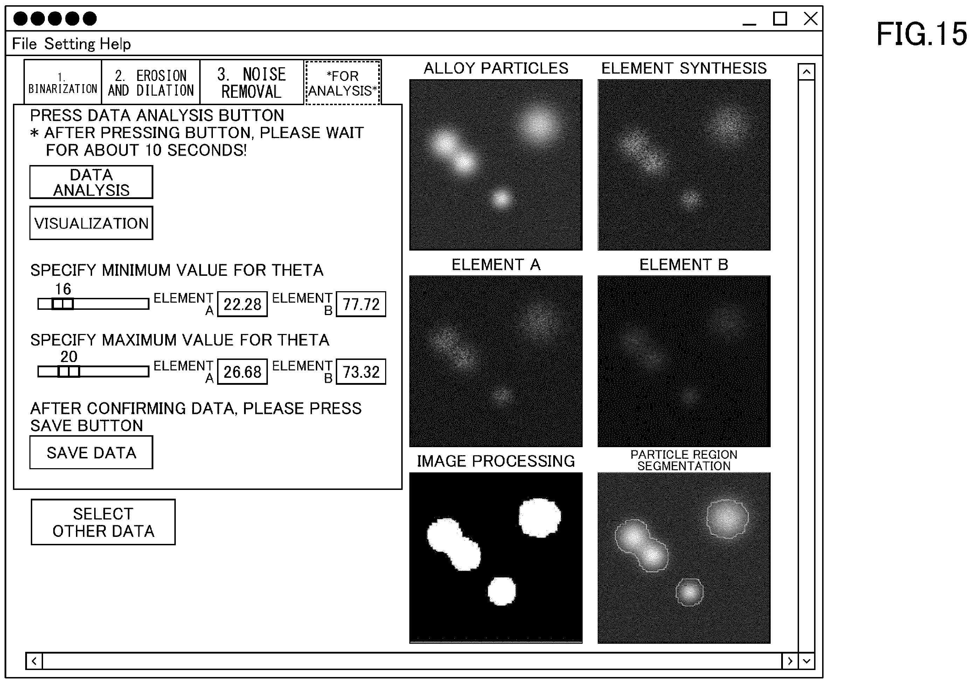

FIG. 15 shows an example of a screen according to one embodiment of the present invention. FIG. 15 shows the screen for the user of the application program to configure settings for analysis.

When the user selects "data analysis" on a parameter setting screen (for analysis), data analysis (in other words, the calculation of the alloy composition) is performed. The composition of the alloy as calculated for each particle is superimposed on a corresponding particle region in the particle region image as shown in the figure, based on the distribution of polar angles in the polar coordinates, where for example, an average value selected by default is displayed overlaid for each particle.

A minimum value of the polar angle θ set by the user in "Specify minimum value for Theta" on the parameter setting screen (for analysis) is used as the range of polar angles θ in a heat map of polar angles θ for pixels (details will be described later). The composition (in the example of Fig. 15, 22.28% is shown for the element A, and 77.72% is shown for the element B) defined by the set minimum value is displayed.

A maximum value of the polar angle θ set by the user in "Specify maximum value of Theta" on the parameter setting screen (for analysis) is used as the range of polar angles θ in the heat map for pixels (details will be described later). The composition (in the example of Fig. 15, 26.68% is shown for the element A, and 73.32% is shown for the element B) defined by the set maximum value is displayed.

When the user selects "Save data" on the parameter setting screen (for analysis), data of an analysis result is saved.

When the user selects "Select other data" on the parameter setting screen (for analysis), data of a different alloy and data of each given element can be re-selected.

### [Analysis Result]

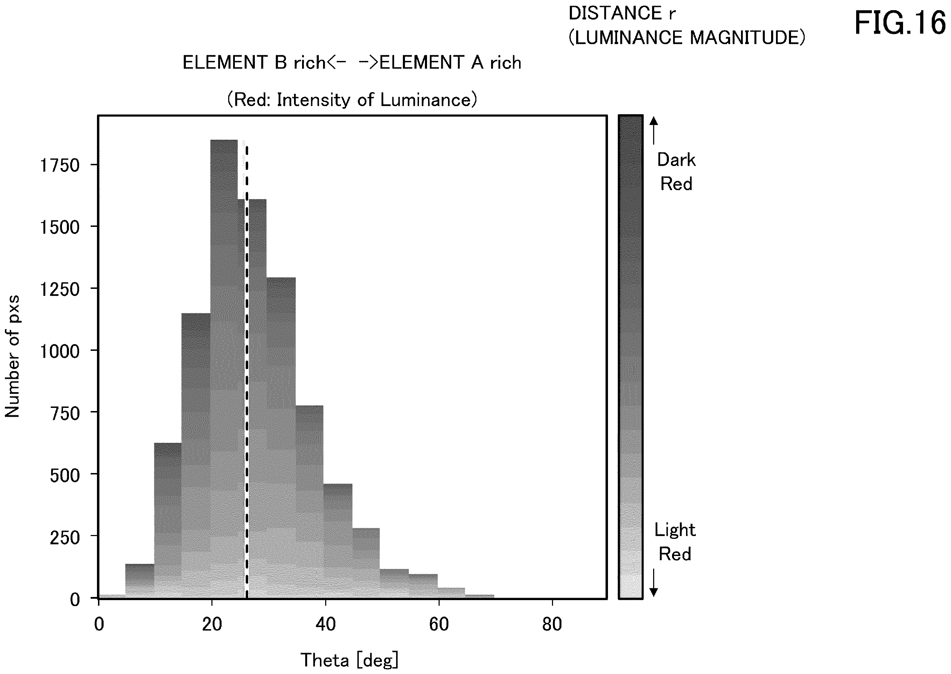

FIG. 16 shows an example of a screen according to one embodiment of the present invention. FIG. 16 shows the screen (which is an example of visualization of the alloy composition) including an analysis result for a selected particle. The horizontal axis expresses the polar angle θ, and the vertical axis expresses the number of pixels for which a target polar angle θ is calculated. A right-side bar in FIG. 16 indicates the intensity of a red color, which is proportional to luminance intensity. With this approach, the polar angles θ for pixels can be defined as a histogram. A statistical value that is calculated based on the histogram corresponds to a bias in the composition of a given alloy particle.

The smaller the polar angle θ, the higher the luminance of the element B in FIG. 16, and the larger the polar angle θ, the higher the luminance of the element A in FIG. 16. In this case, the alloy composition calculation apparatus 10 can provide the distribution of polar angles θ in the polar coordinates (that is, the bias between the element A and the element B), for each alloy particle or for each alloy image.

A dotted line in FIG. 16 indicates the polar angle θ corresponding to one or more setting values (a composition for the alloy data that is selected by the user and input on the initial screen). Ideally, if there is no variation, data clusters around the setting values (the dotted line).

In addition, by indicating the number of pixels by distance from the origin in the polar coordinates (for example, as shown in FIG. 16, each color density is shown separately), a detailed account of r at each polar angle θ is visualized. With this approach, the alloy composition calculation apparatus 10 can provide both the distribution of polar angles θ (that is, the bias between the element A and the element B) in the polar coordinates and the distribution of distances (that is, an amount of each element) from the origin in the polar coordinates, for each alloy particle or each alloy image.

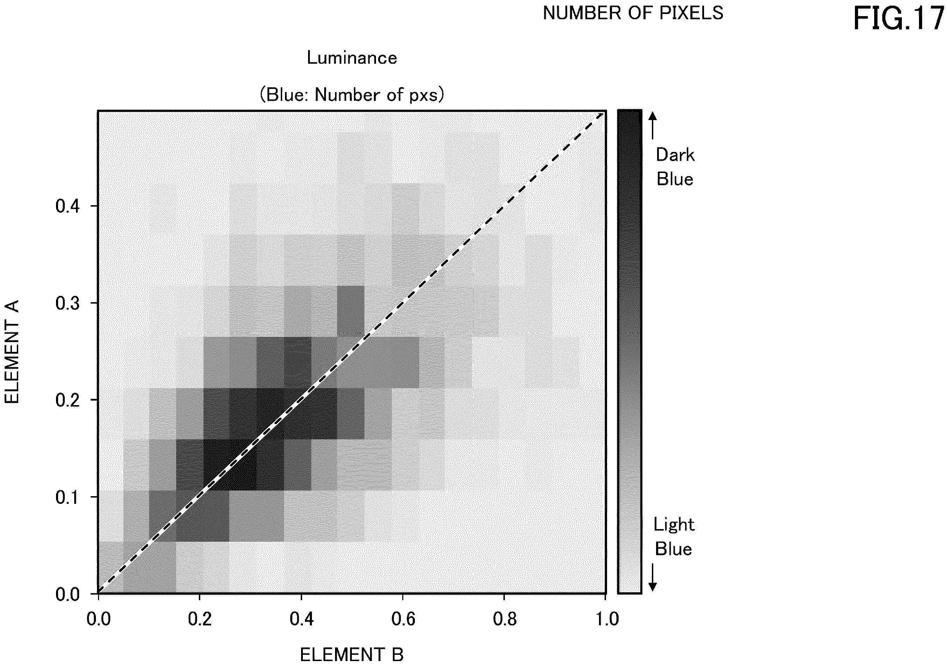

FIG. 17 shows an example of a screen according to one embodiment of the present invention. FIG. 17 shows the screen (which is an example of visualization of the alloy composition) including an analysis result. The vertical axis expresses the luminance of the element image of the element A, and the horizontal axis expresses the luminance of the element image of the element B. A right-side bar in FIG. 17 indicates the intensity of blue color, which is proportional to a number related with corresponding luminance. The number of pixels related with each luminance is displayed in a distinguishable manner (for example, as shown in FIG. 17, each color density is shown separately). With this approach, the luminance of the pixels can be defined as a heat map (in other words, luminance frequency distribution is visualized).

A dotted line (diagonal line) in FIG. 17 indicates the luminance corresponding to one or more setting values (composition for the alloy data that is selected by the user and input on the initial screen). Ideally, if there is no variation, data clusters around the dotted line (diagonal line) in FIG. 17. With this approach, in one embodiment of the present invention, a correlation coefficient between the luminance for the element A and the luminance for the element B can be visualized.

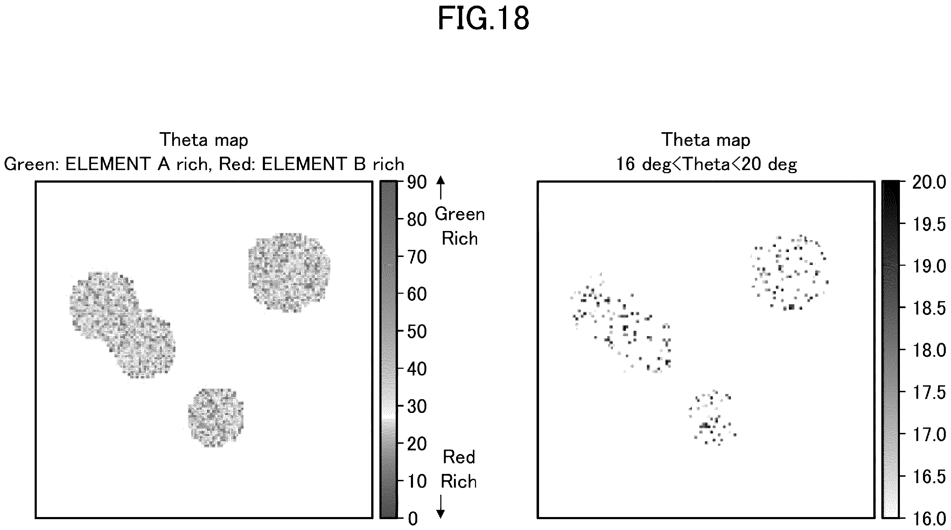

FIG. 18 shows an example of a screen according to one embodiment of the present invention. FIG. 18 shows the screen including an analysis result (which is an example of visualization of the alloy composition). As shown on the left side of FIG. 18, polar angles θ for pixels can be defined as a heat map. Further, as shown on the right side of FIG. 18, a heat map defined in the range of polar angles θ that is entered on the parameter setting screen (for analysis) can be shown in monochrome.

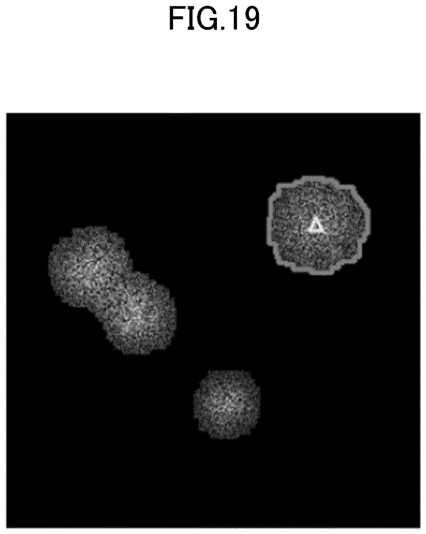

FIG. 19 shows an example of a screen according to one embodiment of the present invention. FIG. 19 shows the screen (which is an example of visualization of the alloy composition) including an analysis result. In this case, a centroid (a center of luminance for a particle region), a luminance centroid for the element A, and a luminance centroid for the element B can be shown. A bias in these elements is identified from a positional relationship (distance and angle) between the centroid (the center of luminance for the particle region) and the luminance centroid for each element.

### <Bias in Alloy Composition>

In one embodiment according to the present invention, the histogram of the polar angles θ for pixels is created, and the bias in the alloy composition is shown by using at least one of a standard deviation for the histogram, a median value for the histogram, an average value for the histogram, or skewness for the histogram. For example, the alloy composition calculation apparatus 10 can provide the bias in the alloy composition by using an area (n m²) of a given image; the standard deviation for the histogram; the median value for the histogram; the skewness for the histogram; average luminance for each element; and a correlation coefficient of luminance for elements in a heat map for pixel luminance. Further, for example, the alloy composition calculation apparatus 10 can provide the bias in the alloy composition by using a positional relationship (distance and angle) between a center of luminance for a particle region and the luminance centroid for each element.

### <Effects>

As described above, according to one embodiment of the present invention, a bias in the alloy composition can be calculated based on the luminance of a given image of each alloy element, for visualization and quantification.

Although the embodiments according to the present invention have been described above in detail, the present invention is not limited to specific embodiment(s) described above, and various modifications and changes can be made within the scope of a gist of the present invention described in the claims.

### [Industrial Usability]

A given alloy composition calculated by the alloy composition calculation apparatus according to the present invention can be used as an indicator suitable for quantitative comparison and contrast of individual manufacturing lots or alloy particles.

This international application claims priority to Japanese Patent Application No. 2022-094234, filed on June 10, 2022, the entire contents of which are hereby incorporated herein by reference.

### Reference Signs List

1 CPU
2 ROM
3 RAM
4 auxiliary storage
5 display
6 control device
7 I/F device
8 drive unit
9 storage medium
10 alloy composition calculation apparatus
11 alloy observation apparatus
12 alloy manufacturing apparatus
20 operator
101 image acquisition unit
102 luminance identifying unit
103 composition calculation unit
104 visualization unit

## Claims

1. An alloy composition calculation apparatus comprising:
an image acquisition unit configured to acquire element images of an alloy;
a luminance identifying unit configured to identify, based on luminance values at identical coordinates in the element images, a point in a Cartesian coordinate system in which axes express luminance of the element images respectively; and
a composition calculation unit configured to calculate a polar angle for the point, in polar coordinates, as a composition of the alloy.

2. The alloy composition calculation apparatus according to claim 1, wherein the composition calculation unit is configured to calculate the composition of the alloy for each particle in the alloy, or for each image of the alloy.

3. The alloy composition calculation apparatus according to claim 1 or 2, wherein the composition calculation unit is configured to calculate the composition of the alloy based on a distribution of polar angles that are calculated in the polar coordinates.

4. The alloy composition calculation apparatus according to claim 3, wherein the composition of the alloy is defined by at least one of a standard deviation, a median value, an average value, or skewness.

5. The alloy composition calculation apparatus according to any one of claims 1 to 4, further comprising:
a visualization unit configured to visualize the composition of the alloy based on polar angles that are calculated in the polar coordinates.

6. The alloy composition calculation apparatus according to claim 5, wherein the visualization unit is configured to provide a distribution of the polar angles in the polar coordinates in a histogram.

7. The alloy composition calculation apparatus according to claim 5, wherein the visualization unit is configured to visualize a distribution of luminance values on each of the element images.

8. A method executed by an alloy composition calculation apparatus, comprising:
acquiring element images of an alloy;
identifying, based on luminance values at identical coordinates in the element images, a point in a Cartesian coordinate system in which axes express luminance of the element images respectively; and
calculating a polar angle for the point, in polar coordinates, as a composition of the alloy.

9. A program causing an alloy composition calculation apparatus to function as:
an image acquisition unit configured to acquire element images of an alloy;
a luminance identifying unit configured to identify, based on luminance values at identical coordinates in the element images, a point in a Cartesian coordinate system in which axes express luminance of the element images respectively; and
a composition calculation unit configured to calculate a polar angle for the point, in polar coordinates, as a composition of the alloy.

10. A system comprising:
an alloy composition calculation apparatus;
an alloy observation apparatus; and
an alloy manufacturing apparatus,
wherein the alloy composition calculation apparatus includes
an image acquisition unit configured to acquire element images of an alloy,
a luminance identifying unit configured to identify, based on luminance values at identical coordinates in the element images, a point in a Cartesian coordinate system in which axes express luminance of the element images respectively, and
a composition calculation unit configured to calculate a polar angle for the point, in polar coordinates, as a composition of the alloy, and
wherein the alloy manufacturing apparatus is configured to manufacture the alloy based on a result of calculation by the alloy composition calculation apparatus.
